# EUROPEAN PATENT APPLICATION

(11) **EP 1 183 991 A2**
(43) Date of publication of application: **06.03.2002**
(21) Application number: 01119742.3
(22) Date of filing: 27.08.2001
(51) Int. Cl.: A61B 1/31

(54) **Surgical/examination anoscope**

(30) Priority: 01.09.2000 IT MO000183
(71) Applicant: Fontana, Paolo, 41100 Modena (IT)
(72) Inventor: Fontana, Paolo, 41100 Modena (IT)

(57) **Abstract**

A surgical/examination anoscope (1) comprising a cylindrical body having an axial cavity (1b) having at least one partially blind straight longitudinal opening on its lateral surface, a solid tip (1a) anatomically contoured for autonomous anal insertion at the insertion end, and, at the opposite open end, a perimetric collar (4) for retaining the perianal epidermis and a maneuvering handle (5) provided with a light source (6); at least the distal transverse internal end wall (7) of the axial cavity is inclined toward the opening and is reflective in order to reflect the light beam from the light source (6) in the cavity through the opening.

## Description

The present invention relates to a surgical/examination anoscope.

So-called anoscopes, i.e., instruments structured specifically to allow medical operators to view the region of the anal orifice by dilating its opening and the initial tract of the rectum, have long been used in the medical field to examine the anorectal region.

These instruments take the form of cylindrical bodies or prong retractors.

In the first case, such cylindrical bodies have diameters calibrated for insertion in the anal canal and are internally crossed by a cavity having an open bottom end; insertion is performed by using appropriately provided cylindrical insertion instruments, which have a rounded end and are removed once the operation has been completed, leaving in place the cylindrical bodies in order to allow operator viewing and optionally the passage of instruments for operating in the viewed regions, for the lighting whereof a light source (lamp or optical fiber) is also provided, the source being integrated in a handle for maneuvering the cylindrical body, which is arranged at the end directed toward the operator.

In the second case, the prongs of retractors (known as Eisenhammer's, Farrand's, Park's retractors) have a rounded shape, so as to define a sort of ogive in a clustered closure configuration, in order to facilitate insertion through the anal orifice, and are provided with means for locking the blades in a divaricated position when they are arranged operatively inside the rectum.

No light source is provided for this type of anoscope.

All viewing or operation occurs, in the first case, through the longitudinal opening, whereas in the second case it can occur by passing between the divaricated blades.

Both the above described anoscope types suffer drawbacks, which make them substantially difficult to use and in some cases even dangerous and painful for the patient. For cylindrical anoscopes, the main problems are the confined viewing and operating field and the unsteady lighting of the region to be viewed that can be achieved.

In the second case, maneuvering the retractors is an extremely delicate procedure which is entrusted to the tactile sensitivity of the medical operator, since no means suitable to limit their opening are provided on such retractors; if the patient has a muscle configuration which is particularly resistant to divarication, or if the operator proceeds with excessive force, the patient may in fact suffer painful traumas which sometimes damage the integrity of the sphincter muscles, with the associated consequences.

Moreover, prong retractors, once positioned and divaricated to allow operation, partially or even fully reduce the visual-operating field formed between the blades due to the natural collapse of the rectal mucous membrane.

Last but not least, prong retractors are usually made of durable material which accordingly can be reused on several patients, albeit every time after appropriate sterilization; if this sterilization is not performed perfectly, it entails the risk of promoting transmission of germs among patients.

The aim of the present invention is to solve the above described problems of the prior art, by providing a surgical/examination anoscope which is sterile and disposable, allows to expose precisely and illuminate better than hitherto possible the operating field, ensures automatic use, i.e. does not need auxiliary tools, is atraumatic in every respect, and allows medical operators better comfort in operation and use with respect to conventional surgical instruments.

This aim and these and other objects which will become better apparent hereinafter are achieved by a surgical/examination anoscope, characterized in that it comprises a cylindrical body provided with an axial cavity and having at least one partially blind straight longitudinal opening on its lateral surface, a solid tip anatomically contoured for autonomous anal insertion at the insertion end, and, at the opposite open end, a perimetric collar for retaining the perianal epidermis and a maneuvering handle provided with a light source, at least the distal transverse internal end wall of said axial cavity being inclined toward said opening and being reflective in order to reflect the light beam from said light source in said cavity through said opening.

Further characteristics and advantages of the present invention will become better apparent from the description of a preferred embodiment of a surgical/examination anoscope, illustrated only by way of non-limitative example in the accompanying drawings, wherein:
Figure 1 is a top view of the surgical/examination anoscope according to a first embodiment of the invention;
Figure 2 is a phantom side view thereof;
Figure 3 is a view of the end of the anoscope that is directed toward the operator;
Figure 4 is a perspective view of the anoscope according to a second embodiment of the invention;
Figures 5 and 6 are corresponding views, taken from above and from the end directed toward the operator, of the anoscope in the embodiment of Figure 4.

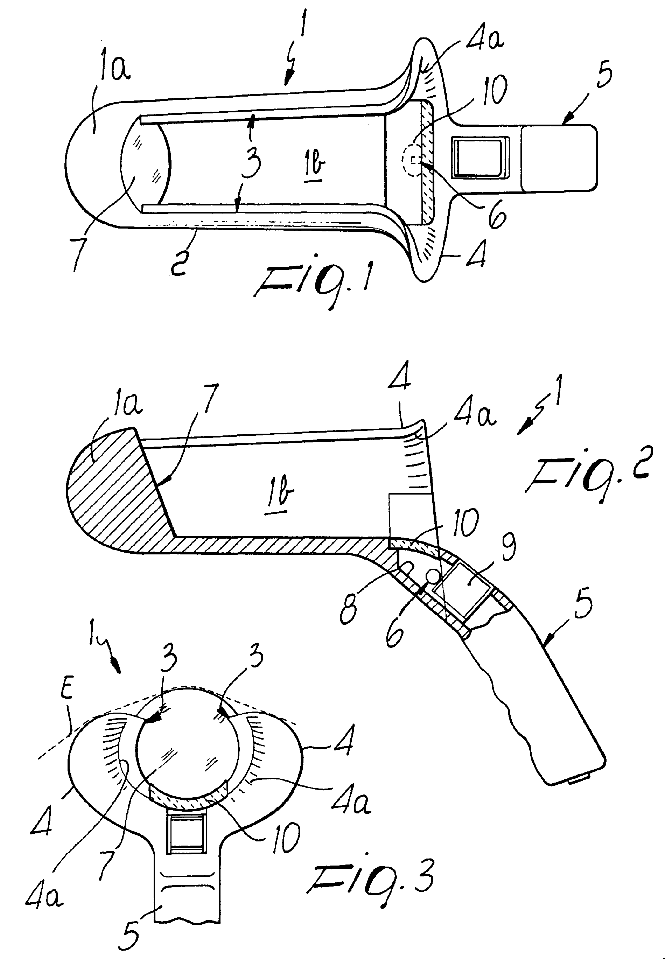

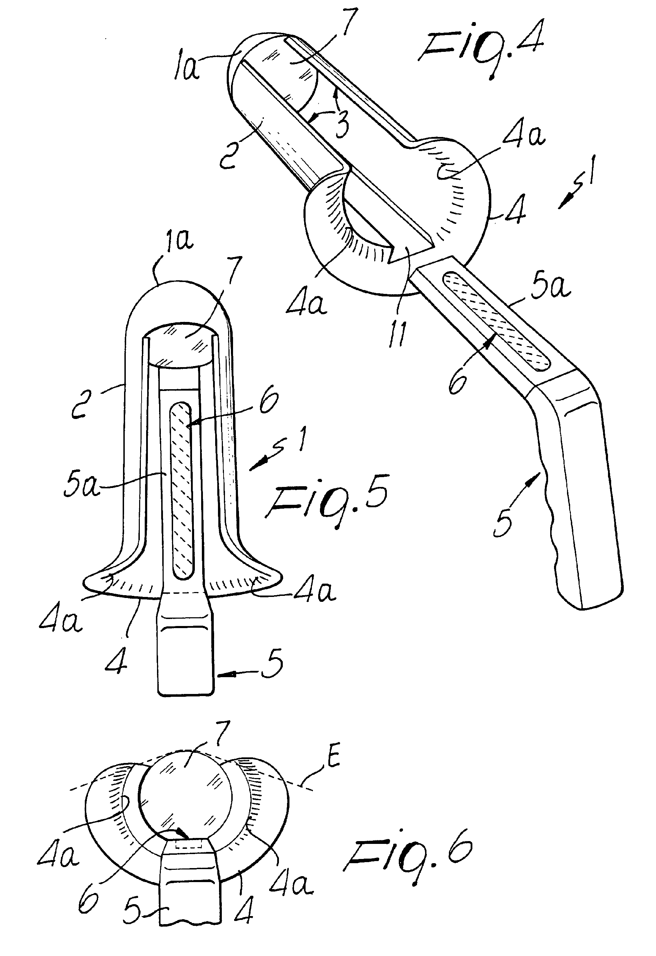

With reference to the figures, the reference numeral 1 designates a surgical/examination anoscope, comprising in a usual manner a cylindrical body 2 which is axially hollow and has, on its lateral surface, at least one straight and partially blind longitudinal opening 3. At the insertion end there is also a solid tip 1a which is anatomically contoured to facilitate insertion of the anoscope in the anal orifice, while at the opposite open end there is a perimetric collar 4, which is adapted to retain the perianal epidermis when the anoscope 1 is inserted, and there is a maneuvering handle 5 provided with a light source 6.

In the anoscope 1, the internal distal transverse wall 7, which in practice constitutes the bottom of an axial cavity 1b defined inside the cylindrical body 2, is inclined toward the opening 3 and its surface is reflective in order to reflect the light beam from the source 6 in the cavity 1b and through said opening 3.

The light source is embedded flush in the wall of the axial cavity 1b, forming a single surface therewith substantially without discontinuities; in particular, in a first embodiment of the anoscope, the light source 6 is located at the inlet of the cavity 1b, in a suitable recess 8 formed at the root of the maneuvering handle 5, and is provided with a connector 9 for connection to a power source and with a transparent cover 10 whose outer surface is, as mentioned, flush with the wall of the axial cavity 1b and practically blends with it.

In a second embodiment of the anoscope 1, the light source 6 is integrated with the handle 5, or more precisely it is arranged inside an elongated element 5a, which is angled with respect to the handle 5 and integral therewith and can be inserted in a bayonet-like fashion in a corresponding seat 11 formed longitudinally in the cavity 1b. The element 5a is advantageously transparent (or has a transparent portion) and has, together with the seat 11, a cross-section whose profiles can be mutually slidingly mated with the profiles of said seat, said profiles preferably having a dovetail shape.

At the end directed toward the operator, the anoscope 1 has a perimetric collar 4, which forms, on the outward face, a profile 4a which is significantly flared and blended with the walls of the axial cavity 1b and is suitable to facilitate insertion and maneuvering, also in a diagonal direction, of conventional medical-surgical operating instruments through said longitudinal opening 3.

Use of the invention is as follows: the surgical/examination anoscope 1 is inserted in the anal orifice of the patient in a conventional manner, facilitated by the anatomically contoured shape of the tip 1a.

Once the anoscope is in position, the operating field is already fully exposed automatically, without additional adjustments: in practice, the tissues are properly distended according to their normal anatomy and the rectal mucous membrane itself does not collapse, accordingly leaving completely free the operating field provided by the anoscope.

The light source 6 emits a light beam, which diffuses inside the axial cavity 1b until it is reflected on the reflective surface of the internal distal transverse wall 7, which by being inclined toward the longitudinal opening 3 directs the reflected beam through it toward the internal walls of the rectal region, lighting it along the entire length of said opening.

The light source 6 can be a simple lamp or optical fibers: in both cases, in the first embodiment of the anoscope 1 the placement can be provided in a recess 8 provided for this purpose in the region for coupling the handle 5 to the cylindrical body 1 and provided with a transparent cover 10, or, in the second embodiment, in an elongated element 5a which protrudes from the handle 5 and acts as a means for coupling it to the body of the anoscope 1, engaging with a bayonet-like coupling in a corresponding seat 11 formed longitudinally in the cavity 1b, to which it is coupled by means of interpenetrating cross-sections having a dovetail profile.

In both embodiments, the light source 6 is, as a whole, flush with the internal surface of the axial cavity 1b in order to eliminate all obstacles to viewing or to the insertion of operating instruments.

The insertion and movement of the instruments for all operations are further facilitated by the significant flaring 4a of the perimetric collar 4, which is normally provided in order to retain the perianal epidermis, shown in dashed lines and designated by the letter E, at the end of the anoscope 1 that is directed toward the operator. The flaring in fact allows to incline the instruments with respect to the longitudinal axis of the cavity 1b by an angle which is significantly greater than possible in known anoscopes.

The anoscope 1 according to the invention is produced in different and calibrated diameters and is strictly single-use and sterile.

In practice it has been observed that the described invention achieves the intended aim and objects.

The invention thus conceived is susceptible of numerous modifications and variations, all of which are within the scope of the inventive concept.

All the details may furthermore be replaced with other technically equivalent elements.

In practice, the materials used, as well as the shapes and the dimensions, may be any according to requirements without thereby abandoning the scope of the protection of the appended claims.

The disclosures in Italian Patent Application No. MO2000A000183 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A surgical/examination anoscope (1), **characterized in that** it comprises a cylindrical body (2) provided with an axial cavity (1b) having at least one partially blind straight longitudinal opening (3) on its lateral surface, a solid tip (1a) anatomically contoured for autonomous anal insertion at the insertion end, and, at the opposite open end, a perimetric collar (4) for retaining the perianal epidermis and a maneuvering handle (5) provided with a light source (6), at least the distal transverse internal end wall (7) of said axial cavity (1b) being inclined toward said opening (3) and being reflective in order to reflect the light beam from said light source (6) in said cavity through said opening.

2. The anoscope according to claim 1, **characterized in that** said light source (6) is embedded flush in the wall of said axial cavity (1b), forming a single surface therewith substantially without discontinuities.

3. The anoscope according to claim 1, **characterized in that** said perimetric collar (4) forms, on the outward face, a flared profile (4a) which is blended with the walls of said axial cavity and is suitable to facilitate the insertion and maneuvering of conventional medical-surgical operating instruments through said longitudinal opening (3).

4. The anoscope according to claims 1 and 3, **characterized in that** said light source (6) is located at the inlet of said axial cavity (1b), within a recess (8) formed at the root of said maneuvering handle (5), a connection (9) to a power source being provided inside said recess (8), said light source (6) being provided with a transparent cover (10) whose outer surface is flush with the wall of said axial cavity (1b).

5. The anoscope according to claims 1 and 2, **characterized in that** said handle (5) forms, in an upward region, an elongated element (5a) which can be inserted and engaged in a bayonet-like fashion in a corresponding seat (11) formed longitudinally in said cavity (1b), said element (5a) being transparent and meant to accommodate said light source (6).

6. The anoscope according to claim 5, **characterized in that** said elongated element (5a) and said corresponding seat (11) have transverse cross-sections whose profiles can be mutually slidingly coupled.

7. The anoscope according to claim 6, **characterized in that** said profiles have a dovetail shape.

8. The anoscope according to the preceding claims, **characterized in that** said cylindrical body can be manufactured with different and calibrated diameters.
